Europäisches Patentamt

European Patent Office

Office européen des brevets

(11) Publication number: **0 150 751**
**B1**

# EUROPEAN PATENT SPECIFICATION

(45) Date of publication of patent specification: **20.04.88**

(21) Application number: **85100319.4**

(22) Date of filing: **15.01.85**

(51) Int. Cl.⁴: **A 61 J 1/06, A 61 M 31/00**

(54) A dispenser containing a liquid or paste-like preparation to be dispensed on a surface area of a human or an animal body.

(30) Priority: **17.01.84 DK 202/84**

(43) Date of publication of application:
**07.08.85 Bulletin 85/32**

(45) Publication of the grant of the patent:
**20.04.88 Bulletin 88/16**

(84) Designated Contracting States:
**AT BE CH DE FR GB IT LI LU NL SE**

(56) References cited:
**CH-A- 630 801**
**DE-A-3 118 580**
**DK-B- 134 308**
**SE-B- 428 758**

(73) Proprietor: **PHARMACIA AS**
**Herredsvejen 2**
**DK-3400 Hillerod (DK)**

(72) Inventor: **Bek, Helge Steen**
**Ebbas Allé 12, Nodebo**
**DK-3480 Fredensborg (DK)**
Inventor: **Jorgensen, Stig**
**Enghavegardsvej 33**
**DK-3400 Hillerod (DK)**

(74) Representative: **Patentanwälte Grünecker, Dr.**
**Kinkeldey, Dr. Stockmair, Dr. Schumann, Jakob,**
**Dr. Bezold, Meister, Hilgers, Dr. Meyer-Plath**
**Maximilianstrasse 58**
**D-8000 München 22 (DE)**

## Description

The present invention relates to a dispenser of the disposable type containing a liquid or paste-like preparation such as a pharmaceutical or a cosmetic preparation, said dispenser comprising a container body having laterally extending and oppositely arranged flat, fin-like parts and an axially extending neck adapted to be fractured at a predetermined position so as to form a discharge opening for said preparation.

DK—B—134.308 discloses such a dispenser, which is made from plastics material and contains a single dose of a pharmaceutical preparation, for example for treating eyes, ears, or the nose. The known dispenser, which contains the pharmaceutical preparation in a sterile condition, is opened immediately before the preparation is to be used by breaking the dispenser neck at a predetermined position, and thereafter substantially the total content of the dispenser is to be used. Such procedure prevents micro-biological contamination and any other contamination of the pharmaceutical preparation which may occur by the use of dispensers containing a plurality of doses of the preparation, which may be dispensed through a drip spout or by means of an associated pipette. When delivered to the final user, a plurality of the described known dispensers may be interconnected by their laterally extending fin-like parts. When the content of one of the dispensers is to be used, the dispenser is separated from the other dispensers, and the dispenser neck is broken at a predetermined position of fracture, whereby an irregular surface of fracture is formed on the neck portion remaining on the dispenser. When the contents of the dispenser is discharged on a surface part to be treated, such as an eye, a nostril, an auditory duct, a wound, or a sore, the irregular surface of fracture may inadvertently be brought into contact with the skin surface or mucous membrane which may be injured.

The present invention provides a dispenser of the above type eliminating the risk that the surface of fracture on the dispenser neck is inadvertently brought into contact with the surface being treated.

The dispenser according to the invention is characterized in that the oppositely arranged fin-like parts extend axially beyond said predetermined position of fracture. These extending fin-like parts may then be brought into contact with the skin surface, the mucous membrane, or the surface area being treated, while the dispenser content is being discharged thereon. Thus, the extending fin-like parts may serve as spacing elements ensuring that the surface of fracture on the dispenser neck is maintained in a position spaced from the surface part on which the dispenser content is discharged.

Eye drop dispensing devices to be used in connection with squeeze bottles containing an ophthalmic liquid are known for example from US—A—2,920,624 and US—A—3,934,590. These known devices comprise a discharge nozzle which is surrounded by a separately formed support or spacing element for preventing the free end of the discharge nozzle from coming into contact with the eyeball. However, these known dispensing devices are adapted to be used in connection with squeeze bottles for containing a plurality of doses of the ophthalmic solution and are of a type which is quite different from that of the dispenser of the present invention.

The neck of the dispenser according to the invention may be adapted to be fractured at said predetermined position by twisting the free end portion of the neck, and the fin-like parts may be shaped so that they may serve as grips for holding the container body while the free end portion of the neck is being twisted. In the absence of such grips it would be necessary to grasp the container body which might thereby be squeezed so that premature and inadvertent discharge of the content could take place.

In principle, the fin-like parts may be fastened to any portion of the dispenser apart from the free end of the neck which is removed when the dispenser is opened. Furthermore, the fin-like part may in principle be separately formed and fastened to the dispenser. In the preferred embodiment, however, the fin-like parts are formed integrally with the neck portion extending between the container body and the predetermined position of fracture and with the adjacent part of the container body.

The free ends of the fin-like parts extending beyond the predetermined position of fracture of the neck should be shaped so as to ensure that they do not hurt the skin surfaces or mucous membranes with which they may come into engagement, while the dispenser content is discharged. In a preferred embodiment of the dispenser according to the invention the flat fin-like parts have convexly curved free ends defining a concavely shaped indentation therebetween, and the predetermined position of fracture may be located adjacent to the bottom part of said indentation.

As mentioned above, the fin-like parts serve as interconnecting parts for interconnecting identical dispensers. Thus, dispensers according to the invention are preferably manufactured, filled, and marketed as a row of identical dispensers which are interconnected by their adjacent fin-like parts. The dispensers may be interconnected along weakening, fracture, or cutting lines so that a dispenser may easily be cut or broken from the other dispensers of the row.

Dispensers according to the invention may be made one by one. However, as mentioned above, they are preferably manufactured as rows of interconnected dispensers made by injection moulding or blow moulding from a suitable plastics material. The dispenser according to the invention may, however, also be made by heat sealing two oppositely arranged films of plastic which may be laminated with metal foils if desired.

The predetermined position of fracture on the container neck may be made by forming the neck with a reduced wall thickness at a predetermined position of fracture. Alternatively, the container neck may be provided with an external indentation or another external weakening.

The invention will now be further described with reference to the drawings, wherein

Fig. 1 is a perspective view of an embodiment of the tube-like dispenser according to the invention,

Fig. 2 is a plan view of a row of interconnected identical dispensers of the type illustrated in Fig. 1, but shown in a reduced scale,

Fig. 3 illustrates how a dispenser of the type shown in Figs. 1 and 2 is opened, and

Fig. 4 illustrates how the content of the opened dispenser may be dripped into an eye.

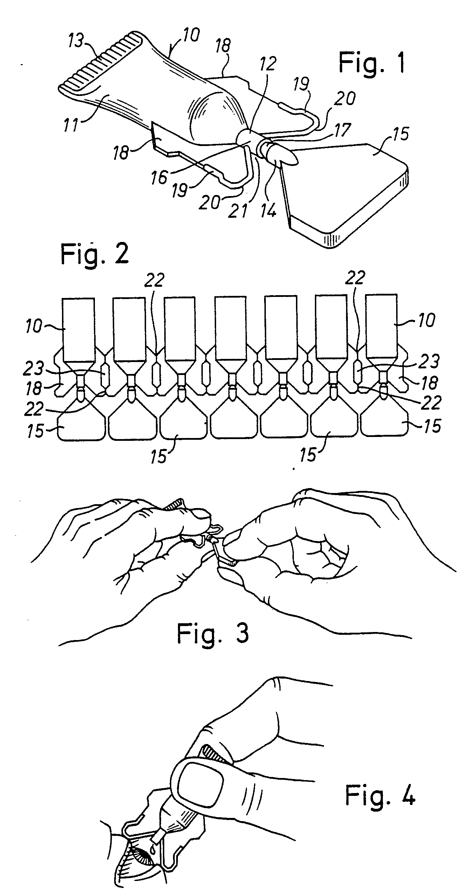

The tube-shaped dispenser or container 10 shown in Fig. 1 comprises a tubular container body 11 merging into a neck 12 at one end and being flattened and closed by means of a heat sealing seam 13 at the other end. The free end portion of the neck 12 is formed as a twist-off closure 14 comprising a flattened finger grip 15. A predetermined position of fracture 17 is formed at a location between the closure 14 and the inner part or root 16 of the neck, and in the embodiment shown in the drawings this position of fracture may be determined by an annular groove formed in the outer surface of the neck. However, in principle, this predetermined position of fracture may be determined by any other type of weakening.

The container or dispenser shown in Fig. 1 also comprises a pair of flat, fin-like parts 18 which extend from opposite sides of the container body 11 and are substantially coplanar with the finger grips 15 of the twist-off closure. In the embodiment shown, the fin-like parts 18 are formed integrally with the adjacent part of the container body 11 and the inner neck part 16. The forward edge portion of each fin-like part 18 is formed as a thickening or bead 19, and the part of the beads 19 which are remote from the predetermined position of fracture 17 form contact surfaces 20 having an axial spacing from the container body exceeding that of the predetermined position of fracture 17. The forward portions of the fin-like parts also have a shape so as to define a concave, such as a V- or a U-shaped notch or cut-out 21 therebetween, and the predetermined position of fracture 17 is located at the bottom of this notch 21.

The container or dispenser shown in Fig. 1 may contain a pharmaceutical or cosmetic preparation such as ear drops, eye drops or a preparation for nasal use, a wound or sore disinfectant, an ointment, eye cosmetics or the like. Such containers or dispensers are advantageously manufactured, filled, closed and marketed with a plurality of identical containers arranged in side-by-side relationship and interconnected, for example as shown in Fig. 2. In Fig. 2 each pair of adjacent identical dispensers or containers 10 have their fin-like parts 18 interconnected along weakening lines 22 which may comprise an elongated cut-out or opening 23 as shown in Fig. 2.

Each single dispenser as that shown in Fig. 1 or a row of dispensers as those shown in Fig. 2 may be made from a suitable plastics material by injection moulding or blow moulding. Alternatively, the tube-shaped dispenser or the interconnected dispensers may be made by heat sealing a pair of oppositely arranged films or foils, which may possibly be vacuum-shaped. Such foils or films may be plastic films or metal foils having a heat sealable plastics material or another heat sealable material applied to their inner sides so as to allow heat sealing of the metal foils. Irrespective of the method of manufacture each single dispenser or container 10 is advantageously formed with a substantially cylindrical container body which is open at the end opposite to the neck 12. The desired liquid or paste-like preparation may then be filled into the dispensers through the open end of the container body which may then be sealed hermetically, for example by means of a transversely extending heat sealing seam as the seam 13 shown in Fig. 1. When a plurality of dispensers or containers interconnected as shown in Fig. 2 has been filled, closed, and possibly sterilized, for example by radiation, the product is ready to be shipped to the final user.

When the user or patient wants to use the contents, such as eye drops, of one of the dispensers 10, the dispenser is cut or separated from the other interconnected containers along one of the weakening lines 22. The dispenser or container may then be opened as illustrated in Fig. 3, which means that the fin-like parts 18 are gripped between the forefinger and thumb of one hand while the fingergrip 15 of the twist-off closure 14 is gripped by the forefinger and thumb of the other hand and twisted in relation to the container body till the neck 12 is broken at the predetermined position of fracture 17.

The eye drops or the eye lotion may now be dripped from the opened dispenser into an eye of the patient as illustrated in Fig. 4 while the curved contact surfaces 20 of the fin-like parts 18 are placed in engagement with the skin surface adjacent to the eye, thus ensuring that the surface of fracture of the neck is maintained suitably spaced from the eyeball whereby any injury to the eyeball by possible projections or roughnesses on the surface of fracture is prevented.

It should be understood that various modifications and changes of the embodiment shown in the drawings could be made within the scope of the invention. As an example, the fin-like parts may have any suitable shape provided that they define contact surfaces which ensure that the predetermined surface of fracture of the neck is kept suitably spaced from the skin surface or mucous membrane or other surface to which the contents of the dispenser is to be applied. As an example, the dispenser may contain a wound disinfectant, and in that case the transverse distance between the contact surfaces 20 of the fin-like parts is preferably increased so that the dispenser content may be applied to greater wound surfaces without bringing the contact surfaces 20 into engagement with the wound.

In the preferred embodiment described above, the fin-like parts have a triple function, because they do not only serve as spacing members, but also as finger grips and as interconnecting fins. It should be understood that within the scope of the present invention it is possible to shape the fin-like parts in such a manner that they serve as spacing elements only and do not have the two other functions mentioned above.

## Claims

1. A dispenser (10) of the disposable type containing a liquid or paste-like preparation to be dispensed on a surface area of a human or an animal body, said dispenser comprising a container body (11) having flat, laterally extending and oppositely arranged fin-like parts (18) and an axially extending neck (12) adapted to be fractured at a predetermined position (17) so as to form a discharge opening for said preparation, characterized in that the oppositely arranged fin-like parts (18) extend axially beyond said predetermined position of fracture (17).

2. A dispenser according to claim 1, characterized in that the neck (12) is adapted to be fractured at said predetermined position by twisting the free end portion (14) of the neck (12), and that the fin-like parts (18) are shaped so that they may serve as grips for holding the container body while the free end portion of the neck (12) is being twisted.

3. A dispenser according to claim 1 or 2, characterized in that the fin-like parts (18) are formed integrally with the neck portion extending between the container body (11) and the predetermined position (17) of fracture and with the adjacent part of the container body (11).

4. A dispenser according to any of the claims 1—3, characterized in that the flat fin-like parts (18) have convexly curved free ends (20) defining a concavely shaped indentation (21) therebetween, and in that the predetermined position of fracture (17) is located adjacent to the bottom part of said indentation.

5. A dispenser according to any of the claims 1—4, characterized in that it is made from a plastics material by injection moulding or blow moulding.

6. An integrally formed dispenser unit comprising a plurality of identical dispensers according to any of the claims 1—5, characterized in that the fin-like parts (18) of adjacent dispensers (10) are interconnected along predetermined fracture or cutting lines (22).

## Patentansprüche

1. Abgabebehälter (10) vom Wegwerftyp, enthaltend ein flüssiges oder pastenartiges Präparat für die Abgabe auf einen Flächenabschnitt eines Menschen- oder Tierkörpers, welcher Abgabebehälter einen Behälterkörper (11) mit flachen, sich quer erstreckenden und gegenüberliegend angeordneten flossenartigen Teilen (18) und einen sich axial erstreckenden Hals (12) aufweist, der dazu bestimmt ist, an einer vorbestimmten Stelle (17) zerbrochen zu werden, um eine Abgabeöffnung für das Präparat zu erzeugen, dadurch gekennzeichnet, daß die gegenüberliegend angeordneten flossenartigen Teile (18) sich in axialer Richtung über die genannte vorbestimmte Bruchstelle (17) hinaus erstrecken.

2. Abgabebehälter nach Anspruch 1, dadurch gekennzeichnet, daß der Hals (12) dazu eingerichtet ist, an der genannten vorbestimmten Stelle durch Verdrehen des freien Endabschnitts (14) des Halses (12) zerbrochen zu werden, und daß die flossenartigen Teile (18) so gestaltet sind, daß sie als Griffe zum Halten des Behälterkörpers dienen können, wenn der freie Endabschnitt des Halses (12) verdreht wird.

3. Abgabebehälter nach Anspruch 1 oder 2, dadurch gekennzeichnet, daß die flossenartigen Teile (18) integral mit dem Halsabschnitt, der sich zwischen dem Behälterkörper (11) und der vorbestimmten Bruchstelle (17) erstreckt, und mit dem benachbarten Teil des Behälterkörpers (11) ausgebildet sind.

4. Abgabebehälter nach einem der Ansprüche 1 bis 3, dadurch gekennzeichnet, daß die flachen flossenartigen Teile (18) konvex gebogene freie Enden (20) haben, die eine konkav gestaltete Einkerbung (21) dazwischen begrenzen, und daß die vorbestimmte Bruchstelle (17) benachbart dem Bodenabschnitt dieser Einkerbung liegt.

5. Abgabebehälter nach einem der Ansprüche 1 bis 4, dadurch gekennzeichnet, daß er aus einem Plastikmaterial durch Spritzgießen oder durch Blasformen hergestellt ist.

6. Integral hergestellte Abgabebehältereinheit, enthaltend mehrere identische Abgabebehälter nach einem der Ansprüche 1 bis 5, dadurch gekennzeichnet, daß die flossenartigen Teile (18) benachbarter Abgabebehälter (10) längs vorbestimmter Bruch- oder Schneidlinien (22) miteinander verbunden sind.

## Revendications

1. Emballage distributeur (10) du type jetable contenant une préparation liquide ou pâteuse à distribuer sur une zone de surface d'un corps humain ou d'un corps animal, ledit emballage distributeur comportant un corps de récipient (11) présentant des parties plates (18) ressemblant à des ailettes, s'étendant latéralement et disposées en face l'une de l'autre ainsi qu'un col (12) s'étendant axialement et prévu pour être brisé en une position prédéterminée (17) de façon à former une ouverture d'expulsion pour ladite préparation, caractérisé en ce que les parties (18) ressemblant à des ailettes et disposées en face l'une de l'autre s'étendent axialement au-delà de la position prédéterminée de brisure (17).

2. Emballage distributeur selon la revendication 1, caractérisé en ce que le col (12) est prévu pour être brisé en ladite position prédéterminée par torsion de la portion d'extrémité libre du col (12); et en ce que les parties (18) ressemblant à des ailettes ont une forme telle qu'elles peuvent servir comme prises pour tenir le corps du récipient pendant que l'on tord la portion d'extrémité libre du col (12).

3. Emballage distributeur selon la revendication 1 ou 2, caractérisé en ce que les parties ressemblant à des ailettes (18) sont venues de forme d'une pièce avec la portion du col qui s'étend entre le corps du récipient (11) et la position prédéterminée (17) de brisure, ainsi qu'avec la partie, voisine, du corps du récipient (11).

4. Emballage distributeur selon l'une quelconque des revendications 1—3, caractérisé en ce que les parties ressemblant à des ailettes (18) pré- - sentent des extrémités libres (20) en courbe convexe définissant entre elles un creux de forme concave (21); et en ce que la position prédéterminée de brisure (17) est située près de la partie inférieure dudit creux.

5. Emballage distributeur selon l'une quelconque des revendications 1—4, caractérisé en ce qu'il est fabriqué en un matériau plastique par moulage par injection ou par moulage par soufflage.

6. Ensemble d'emballages distributeurs formés d'une pièce, comportant une pluralité d'emballages distributeurs identiques selon l'une quelconque des revendications 1—5, caractérisé en ce que les parties (18) ressemblant à des ailettes des emballages distributeurs voisins (10) sont interconnectées le long de lignes prédéterminées de brisure ou de coupe (22).

Fig. 1

Fig. 2

Fig. 3

Fig. 4